# EUROPEAN PATENT APPLICATION

(11) **EP 3 381 442 A1**
(43) Date of publication of application: **03.10.2018**
(21) Application number: 17164108.7
(22) Date of filing: 31.03.2017
(51) Int. Cl.: A61K 8/97, A61Q 19/08, A61Q 17/00

(54) **COSMETIC COMPOSITIONS**

(71) Applicant: DSM IP Assets B.V., 6411 TE Heerlen (NL)
(72) Inventor: Campiche, Remo, 4303 Kaiseraugst (CH); Gempeler, Mathias, 4303 Kaiseraugst (CH); Schuetz, Rolf, 4303 Kaiseraugst (CH)
(74) Representative: Berg, Katja

(57) **Abstract**

The present invention relates to cosmetic compositions comprising an extract of scenedesmus rubescens and a cosmetically acceptable carrier. Furthermore, the invention relates to a method for the protection of human skin against light damage such as in particular to protect human skin from damage by exposure to blue light.

## Description

The present invention relates to cosmetic compositions comprising an extract of scenedesmus rubescens and a cosmetically acceptable carrier. Furthermore, the invention relates to a method for the protection of human skin against electromagnetic radiation, such as in particular to visible light damages.

The deleterious effects of skin exposure to ultraviolet (UV) radiation (both UVA and UVB) are well known. The UV radiation is categorized into three regions, UVC, UVB and UVA. Most of the UVC is filtered by ozone layer and high energy low wavelength UVB get absorbed in the upper layers of skin, i.e. epidermal region, while UVA penetrates little deeper into dermal regions of the skin. So far, most of the studies on skin exposure to light were concentrated on the role of UV irradiation due to its high energy, photo reactivity and its associated damage to the skin while the role of visible light has been less extensively investigated. In recent times, however, the adverse effects of visible light on skin has become more and more apparent.

Visible light is the region of light with 400-700 nm in the electromagnetic spectrum. Blue light is the portion of the electromagnetic spectrum in the visible region with wavelengths ranging from 400-500 nm. The wavelengths of blue light are close to UVA spectrum (315-400 nm) and the blue region of the visible spectrum is particularly important because it has a relatively high energy and at the same time longer wavelengths that can thus penetrate tissue deeper than UV light. It has recently been shown that blue light induces Reactive Oxygen Species (ROS) and Matrix-Degrading Enzymes in the skin which may lead inter alia to mitochondrial DNA damage and collagen degradation. Additionally, ROS generated by UV and blue light can cause further damage to proteins by oxidation of lysine, arginine, proline, and threonine residues. In addition, carbonyl groups may be introduced into proteins by reactions with aldehydes (4-hydroxy-2-nonenal, malondialdehyde) produced during lipid peroxidation or with reactive carbonyl derivatives generated as a consequence of the reaction of reducing sugars or their oxidation products with lysine residues of proteins. Both ROS and carbonyl groups on proteins (carbonylated proteins) can easily be measured in cells and tissue by a person skilled in the art and serve as markers for cell damage.

Based on the more and more apparent adverse effects of electromagnetic radiation such as in particular visible light, there is an ongoing need for agents, which can protect the skin against electromagnetic radiation such as in particular visible light including blue light.

Surprisingly, it has been found that an extract of the microalgae *Scenedesmus Rubescens* is suitable to protect the skin from the adverse effects of electromagnetic radiation such as in particular of blue light.

Thus, in one aspect, the invention relates to cosmetic compositions comprising a scenedesmus rubescens extract and a cosmetically acceptable carrier.

In another aspect, the present invention relates to a method of protecting human skin against damage upon exposure to electromagnetic radiation, said method comprising the step of applying a cosmetic composition containing an effective amount of a scenedesmus rubescens extract to the skin.

In a further embodiment, the present invention also relates to a method of reducing the generation of reactive oxygen species and/ or the formation of carbonylated proteins in humans when exposed to blue light, said method comprising the step of applying a cosmetic composition containing an effective amount of a scenedesmus rubescens extract to the skin.

In a still further embodiment, the present invention relates to the use of a scenedesmus rubescens extract to protect human skin against the adverse effects of electromagnetic radiation, such as in particular of visible light and most in particular of blue light.

In a particular advantageous embodiment, the present invention relates to the use of a scenedesmus rubescens extract to protect human skin against the formation of reactive oxygen species, of sunburn cells and/ or of carbonylated proteins as well as the induction of MMP degrading enzymes.

The term 'electromagnetic radiation' preferably refers to electromagnetic radiation between 290nm and 3.0µm. Such radiation can be emitted from natural sources as well as from artificial devices such as electronic displays. More preferably, in all embodiments of the present invention, the term 'electromagnetic radiation' refers to is visible light, most preferably blue light.

The term 'adverse effects of light' as used herein encompasses in particular the generation of reactive species by electromagnetic irradiation such as in particular the formation of reactive oxygen species, reactive nitrogen species (RNS) and reactive carbonyl species (RCS). Reactive species include radical and non-radical compounds such as nitroxyl-, carbonyl-, hydroxy- or oxyl-radicals, peroxylradicals, peroxides, superoxideradicalanion, hydrogenperoxide, singlet oxygen, lipoperoxides such as squaleneperoxides, ozone, nitrogene oxydes and NO-radical. The term 'adverse effects of light' also includes the formation of sunburn cells and/ or of carbonylated proteins as well as the induction of MMP degrading enzymes.

The term 'scenedesmus rubescens extract' as used herein generally refers to an extract of the biotechnologically produced microalgae *Scenedesmus Rubescens.* Such an extract can be obtained by standard methods in the art e.g. by cultivation of the respective algae in a photo-bioreactor, followed by harvesting the cells resulting in a *Scenedesmus Rubescens* biomass. Afterwards the biomass is extracted with hot water. The thus obtained crude extract is subsequently centrifuged and ultrafiltrated.

In a particular advantageous embodiment, the scenedesmus rubescens extract according to the present invention is obtained by a method comprising the steps of cultivating a strain of scenedesmus rubescens in a photo-bioreactor, followed by extraction of the biomass with hot water and subsequent centrifugation and ultrafiltration of the thus obtained crude extract.

The scenedesmus rubescens extract according to the present invention can be used in dried form or in the form of an aqueous extract with a known solid content (i.e. dry matter also known as residue on drying). The solid content in the aqueous extracts according to the present invention can easily be adjusted by standard methods in the art either by evaporation of or by dilution with water.

Preferably, in all embodiments of the present invention an aqueous scenedesmus rubescens extract with a solid content selected in the range of 0.5 to 10 wt.-%, preferably in the range of 1 to 5 wt.-%, based on the total weight of the extract is used. In addition, the pH of such an aqueous extract is preferably selected in the range of 3 to 6, most preferably in the range of 4.5 to 5.5.

In a particular advantageous embodiment, the aqueous extracts according to the present invention furthermore contain at least one preservative. Particular suitable preservatives according to the present invention are selected from the group consisting of phenoxyethanol, sodium benzoate and potassium sorbate as well as mixtures thereof. Most preferably, the preservative used in the aqueous extracts according to the present invention is phenoxyethanol.

A particularly suitable scenedesmus rubescens extract according to the present invention is an aqueous extract having a solid content selected in the range of 1-5 wt.-% and which further comprises an amount of phenoxyethanol selected in the range of 0.1 to 1 wt.-% and which exhibits a pH in the range of 4.5-5.5. Such an extract is e.g. available from DSM Nutritional Products under the tradename PEPHA®-AGE.

The term 'an effective amount' as used herein refers to an amount necessary to obtain the physiological effect. The physiological effect may be achieved by one application dose or by repeated applications. The dosage administered may, of course, vary depending upon known factors, such as the physiological characteristics of the cosmetic composition comprising the respective extract and its mode and route of administration; the age, the nature and extent of the symptoms; the kind of concurrent treatment; the frequency of treatment; and the effect desired and can be adjusted by a person skilled in the art.

The total amount of the scenedesmus rubescens extract (based on dry matter) in the cosmetic compositions according to the present invention is generally selected in the range of 0.001 to 1 wt.-%, preferably in the range of 0.01 to 0.75 wt.-%, such as most preferably in the range of 0.025 to 0.5 wt.-% and in particular in the range of 0.025 to 0.1 wt.-% based on the total weight of the cosmetic composition.

The term 'cosmetic composition' as used herein refers to compositions, which are used to treat, care for or improve the appearance of the skin and/or the scalp. Particular advantageous cosmetic compositions according to the present invention are skin care preparations.

The term 'cosmetically acceptable carrier' (also referred to herein as carrier) refers to all vehicles/ carriers conventionally used in cosmetic compositions, i.e. which are suitable for topical application to the keratinous tissue, have good aesthetic properties, are compatible with the actives present in the composition, and will not cause any unreasonable safety or toxicity concerns. Such carriers are well-known to one of ordinary skill in the art.

The exact amount of carrier will depend upon the actual level of the scenedesmus rubescens extract and of any other optional ingredients that one of ordinary skill in the art would classify as distinct from the carrier (e.g., other active ingredients).

In a particular advantageous embodiment, the compositions according to the present invention further comprise at least one UV-filter substance.

Suitable UV-filter substances according to the invention are UVA, UVB and/ or broadspectrum UV-filter substances which are or can be used as cosmetically acceptable UVA, UVB or broadspectrum UV-filter substances. Such UV-filter substances are e.g. listed in the CTFA Cosmetic Ingredient Handbook or in the Regulation (EC) No 1223/2009 of the European Parliament and of the Council.

Preferred UVB filter substances to be incorporated into the compositions according to the invention encompass polysilicone-15, phenylbenzimidazol sulfonic acid, octocrylene, ethylhexyl methoxycinnamate, ethylhexyl salicylate and/ or homosalate.

Preferred broadband UV-filter substances encompass bis-ethylhexyloxyphenol methoxyphenyl triazine, 2-hydroxy-4-methoxy-benzophenon, methylene bis-benzotriazolyl tetramethylbutylphenol and/ or titanium dioxide.

Preferred UVA-filter substances encompass butyl methoxydibenzoylmethane, diethylamino hydroxybenzoyl hexyl benzoate, 2,4-bis-[5-1(dimethylpropyl)benzoxazol-2-yl-(4-phenyl)-imino]-6-(2-ethylhexyl)-imino-1,3,5-triazine, zinc oxide and/ or disodium phenyl dibenzimidazole tetrasulfonate.

In an advantageous embodiment, the compositions according to the present invention further comprise at least 2, more preferably at least 3, most preferably at least 4 different UV-filter substances. It is further advantageous, if at least one UVB filter substance and/ or broadband filter substance and at least one UVA-filter substance is present.

In a particular advantageous embodiment, the at least one UV-filter substance present in compositions according to the invention is selected from the group consisting of polysilicone-15, phenylbenzimidazol sulfonic acid, octocrylene, ethylhexyl methoxycinnamate, ethylhexyl salicylate, homosalate, bis-ethylhexyloxyphenol methoxyphenyl triazine, methylene bis-benzotriazolyl tetramethylbutylphenol, titanium dioxide, butyl methoxydibenzoylmethane, diethylamino hydroxybenzoyl hexyl benzoate, disodium phenyl dibenzimidazole tetrasulfonate as well as mixtures thereof.

In an even more preferred embodiment, the at least one UV-filter substance is selected from the group consisting of bis-ethylhexyloxyphenol methoxyphenyl triazine, polysilicone-15, butyl methoxydibenzoylmethane, ethylhexyl salicylate, octocrylene, homosalate, methylene bis-benzotriazolyl tetramethylbutylphenol, titanium dioxide as well as mixtures thereof.

In all embodiments of the invention, the amount of the UV-filter substances (i.e. the sum of all UV-filter substances present in compositions according to the present invention is preferably selected in the range of 1 to 40 wt.-%, more preferably in the range of 5 to 35 wt.-% and most preferably in the range of 10 to 30 wt.-% based on the total weight of the topical sunscreen emulsion.

In an even more preferred embodiment, the compositions according to the invention comprise a mixture of UV-filters selected from the group of bis-ethylhexyloxyphenol methoxyphenyl triazine, polysilicone-15, butyl methoxydibenzoylmethane, ethylhexyl salicylate, octocrylene, methylene bis-benzotriazolyl tetramethylbutylphenoland titanium dioxide as sole UV-filter substances. In this case the total amount of the UV-filter substances preferably sums up to 15 to 25 wt%.

In an advantageous embodiment, the cosmetic compositions according to the present invention comprise from about 50% to about 99%, preferably from about 60% to about 98%, more preferably from about 70% to about 98%, such as in particular from about 80% to about 95% of a carrier, based on the total weight of the cosmetic composition.

In an advantageous embodiment, the carrier consists furthermore of at least 40 wt.-%, more preferably of at least 50 wt.-%, most preferably of at least 55 wt.-% of water, such as in particular of about 55 to about 90 wt.-% of water.

The compositions of the invention (including the carrier) may comprise conventional adjuvants and additives, such as preservatives/antioxidants, fatty substances/oils, organic solvents, silicones, thickeners, softeners, emulsifiers, antifoaming agents, aesthetic components such as fragrances, surfactants, fillers, anionic, cationic, nonionic or amphoteric polymers or mixtures thereof, propellants, acidifying or basifying agents, dyes, colorings/colorants, abrasives, absorbents, chelating agents and/ or sequestering agents, essential oils, skin sensates, astringents, pigments or any other ingredients usually formulated into such compositions.

In accordance with the present invention, the compositions according to the invention may also comprise further cosmetically active ingredients conventionally used in cosmetic compositions. Exemplary active ingredients encompass skin lightening agents; UV-filters, agents for the treatment of hyperpigmentation; agents for the prevention or reduction of inflammation; firming, moisturizing, soothing, and/ or energizing agents as well as agents to improve elasticity and skin barrier.

Examples of cosmetic excipients, diluents, adjuvants, additives as well as active ingredients commonly used in the skin care industry which are suitable for use in the cosmetic compositions of the present invention are for example described in the International Cosmetic Ingredient Dictionary & Handbook by Personal Care Product Council (http://www.personalcarecouncil.org/), accessible by the online INFO BASE (http://online.personalcarecouncil.org/jsp/Home.jsp), without being limited thereto.

The necessary amounts of the active ingredients as well as the excipients, diluents, adjuvants, additives etc. can, based on the desired product form and application, easily be determined by the skilled person. The additional ingredients can either be added to the oily phase, the aqueous phase or separately as deemed appropriate.

The cosmetically active ingredients useful herein can in some instances provide more than one benefit or operate via more than one mode of action.

Of course, one skilled in this art will take care to select the above mentioned optional additional ingredients, adjuvants, diluents and additives and/or their amounts such that the advantageous properties intrinsically associated with the combination in accordance with the invention are not, or not substantially, detrimentally affected by the envisaged addition or additions.

The cosmetic compositions according to the present invention can be in a wide variety of forms. Non limiting examples include simple solutions (e.g. aqueous, organic solvent, or oil based), emulsion or micro emulsion (in particular of oil-in-water (O/W) or water-in-oil (W/O) type, silicone-in-water (Si/W) or water-in-silicone (W/Si) type, PIT-emulsion, multiple emulsion (e.g. of oil-in-water-in oil (O/W/O) or water-in-oil-in-water (W/O/W) type) or pickering emulsions), as well as solid forms (e.g. hydrogels, alcoholic gels, lipogels, sticks, flowable solids, or amorphous materials).

These product forms may be used for a number of applications, including, but not limited to, hand and body lotions, facial moisturizers, anti-ageing preparations, make-ups including foundations, and the like. Any additional components required to formulate such products vary with product type and can be routinely chosen by one skilled in the art.

If the composition is an emulsion, such as in particular an O/W-, W/O-, Si/W-, W/Si-, O/W/O-, W/O/W- or a pickering emulsion, then the amount of the oily phase present in such cosmetic emulsions is preferably at least 10 wt.-%, such as in the range of 10 to 60 wt.-%, preferably in the range of 15 to 50 wt.-%, most preferably in the range of 15 to 40 wt.-%, based on the total weight of the composition.

In one embodiment, the compositions according to the present invention are advantageously in the form of an oil-in-water (O/W) emulsion comprising an oily phase dispersed in an aqueous phase in the presence of an O/W emulsifier. The preparation of such O/W emulsions is well known to a person skilled in the art.

If the composition according to the invention is an O/W emulsion, then it contains advantageously at least one O/W- or Si/W-emulsifier selected from the list of, glyceryl stearate citrate, glyceryl stearate SE (self-emulsifying), stearic acid, salts of stearic acid, polyglyceryl-3-methylglycosedistearate. Further suitable emulsifiers are phosphate esters and the salts thereof such as cetyl phosphate (e.g. as Amphisol® A from DSM Nutritional Products Ltd.), diethanolamine cetyl phosphate (e.g. as Amphisol® DEA from DSM Nutritional Products Ltd.), potassium cetyl phosphate (e.g. as Amphisol® K from DSM Nutritional Products Ltd.), sodium cetearylsulfate, sodium glyceryl oleate phosphate, hydrogenated vegetable glycerides phosphate and mixtures thereof. Further suitable emulsifiers are polyalkylene glycol ethers, sorbitan oleate, sorbitan sesquioleate, sorbitan isostearate, sorbitan trioleate, cetearyl glucoside, lauryl glucoside, decyl glucoside, sodium stearoyl glutamate, sucrose polystearate and hydrated polyisobutene. Furthermore, one or more synthetic polymers may be used as an emulsifier. For example, PVP eicosene copolymer, acrylates/C10-30 alkyl acrylate crosspolymer, and mixtures thereof.

The at least one O/W, respectively Si/W emulsifier is preferably used in an amount of 0.5 to 10 wt. %, in particular in the range of 0.5 to 6 wt.-%, such as more in particular in the range of 0.5 to 5 wt.-%, such as most in particular in the range of 1 to 4 wt.-%, based on the total weight of the composition.

Particular suitable O/W emulsifiers to be used in the compositions according to the invention encompass phosphate ester emulsifiers such as advantageously 8-10 alkyl ethyl phosphate, C9-15 alkyl phosphate, ceteareth-2 phosphate, ceteareth-5 phosphate, ceteth-8 phosphate, ceteth-10 phosphate, cetyl phosphate, C6-10 pareth-4 phosphate, C12-15 pareth-2 phosphate, C12-15 pareth-3 phosphate, DEA-ceteareth-2 phosphate, DEA-cetyl phosphate, DEA-oleth-3 phosphate, potassium cetyl phosphate, deceth-4 phosphate, deceth-6 phosphate and trilaureth-4 phosphate as well as polyalkylene glycol ethers such as in particular polyethylene stearyl ethers such as Steareth-2 and Steareth 21.

A particular suitable class of O/W emulsifier to be used in the compositions according to the invention are the cetyl phosphates such as in a particular potassium cetyl phosphate available at DSM Nutritional Products under the tradename Amphisol K.

In one particular embodiment, the invention relates to compositions with all the definitions and preferences given herein in the form of O/W emulsions comprising an oily phase dispersed in an aqueous phase in the presence of at least one O/W emulsifier wherein the at least one O/W emulsifier is potassium cetyl phosphate.

The cosmetic compositions according to the present invention advantageously comprise a preservative. Particular suitable preservatives in all embodiments of the present invention are phenoxyethanol and ethylhexylglycerin as well as mixtures thereof. When present, the preservative is preferably used in an amount of 0.1 to 2 wt.-%, more preferably in an amount of 0.5 to 1.5 wt.-%, based on the total weight of the composition.

The compositions according to the invention in general have a pH in the range of 3 to 10, preferably a pH in the range of 4 to 8 and most preferably a pH in the range of 5 to 8. The pH can easily be adjusted as desired with suitable acids, such as e.g. citric acid, or bases, such as sodium hydroxide (e.g. as aqueous solution), triethanolamine (TEA Care), Tromethamine (Trizma Base) and Aminomethyl Propanol (AMP-Ultra PC 2000), according to standard methods in the art.

The amount of the compositions to be applied to the skin is not critical and can easily be adjusted by a person skilled in the art. Preferably the amount is selected in the range of 0.1 to 3 mg/ cm² skin, such as preferably in the range of 0.1 to 2 mg/ cm² skin and most preferably in the range of 0.5 to 2 mg / cm² skin.

The following examples are provided to further illustrate the compositions and effects of the present invention. These examples are illustrative only and are not intended to limit the scope of the invention in any way.

### Example 1: Preparation of scenedesmus rubescens extracts

Scenedesmus rubescens biomass obtained via cultivation of a strain of the microalgae Scenedesmus rubescens in a photo-bioreactor was suspended in water, followed by heating the resulting suspension to about 100°C for about 2 hours. Then, the liquid and solid phases were separated by centrifugation and the liquid phase was further purified by ultrafiltration. The solid content of the thus obtained aqueous extract was determined and, if necessary, adjusted by addition with water to about 2.5 wt.-%. If furthermore necessary, the pH was adjusted with hydrochloric acid to about 5. The aqueous scenedesmus rubescens extract was then formulated with 0.1 wt.-% of Phenoxyethanol, microfiltrated and filled in a storage containers

For the subsequent experiments, a 1 wt.-% (i.e. 0.025 wt.-% solid content), respectively a 3 wt.-% (i.e. 0.075 wt.-% solid content) dilution of the above-mentioned extract in DSMO was prepared.

### Example 2: Protection against blue light induced reactive oxygen species ex-vivo

The ROS detection probe DCFH-DA has been applied in the culture medium for 30 minutes before the irradiation. After changing the medium, the respective scenedesmus rubescens extract in DSMO was applied topically onto the skin tissue and then blue light (100J/cm²) irradiation (380-470nm, max at 420nm, UV436 HF b Waldmann GmBH Co) was performed, followed again by topical application of the respective scenedesmus rubescens extract in DSMO. After 24h post-irradiation, the skin samples are harvested, cryo-fixed and cut at the cryostat for subsequent image acquisition and analysis. For each image the upper dermis has been analyzed by evaluating the fluorescence through Image-J application (NIH, USA). The obtained value has been normalized upon the dimension of the selected area.

| **Test samples** | **ROS** | **SEM*** |
|---|---|---|
| Vehicle control (DMSO), no Blue light | 4 % | 0.8 % |
| Vehicle control (DMSO), Blue light | 100 % | 11 % |
| 1 wt.-% Scenedesmus rubescens extract in DMSO | 68 % | 12 % |
| 3 wt.-% Scenedesmus rubescens extract in DMSO | 65 % | 9 % |

| | | |
|---|---|---|
| * standard error of the mean | | |

As can be retrieved from table 1, the treatment with an extract according to the present invention significantly reduces the formation of ROS upon irradiation with Blue light.

### Example 3: Evaluation of blue light induced carbonylated proteins ex-vivo

The respective scenedesmus rubescens extract in DSMO was topically applied to skin tissue before and right after irradiation with blue light. Extract application was renewed after 24h. Carbonylated proteins were extracted from tissues 48h post-irradiation with blue light (380-470nm, max at 420nm, UV436 HF by Waldmann GmBH Co). After harvesting, the frozen skin samples have been weighted and minced. The skin samples for each tested condition have been pooled and processed together by homogenization in cold extraction buffer. Total cell lysates have been recovered by centrifugation. Carbonylated proteins in skin samples have been determined by "OxyBlot Protein Oxidation Detection Kit" (Millipore, #S7150) according to the manufacturer's instructions by western blot and chemiluminescence detection. The extent of the carbonyl content obtained for each sample have been normalized on actin protein signal band.

| **Test samples** | **Carbonylated proteins** | **SEM*** |
|---|---|---|
| Vehicle control (DMSO), no Blue light | 31 % | 6 % |
| Vehicle control (DMSO), Blue light | 100 % | 0 % |
| 1 wt.-% Scenedesmus rubescens extract in DMSO | 39 % | 12 % |
| 3 wt.-% Scenedesmus rubescens extract in DMSO | 41 % | 12 % |

| | | |
|---|---|---|
| * standard error of the mean | | |

As can be retrieved from table 2, the treatment with an extract according to the present invention significantly reduces the formation of carbonylated proteins upon irradiation with Blue light.

### Example 4: After Sun Cooling Spray

| **Phase** | **INCI Name** | **Wt.-%** |
|---|---|---|
| A | Water | Ad 100 |
| | Microcrystalline Cellulose, Cellulose Gum | 1.5 |
| | Allantoin | 0.1 |
| | Phenoxyethanol, Ethylhexylglycerin | 1.00 |
| B | Xanthan gum | 0.15 |
| | Propanediol | 5.000 |
| C | Alcohol (Ethanol) | 5.00 |
| D | Polyglyceryl-10 laurate, Aqua, Citric acid | 2.00 |
| | Extract according to example 1 | 1.00 |
| | Cl 42090, Aqua | 0.50 |
| E | Parfum | 0.20 |
| | Butylene glycol dicaprylate/dicaprate | 3.50 |
| | Tocopheryl acetate | 0.50 |
| | Menthyl ethylamido oxalate | 1.00 |
| F | Citric acid, Aqua* | q.s. |

| | | |
|---|---|---|
| * to adjust the pH if necessary to pH of about 6. | | |

### Example 5: In and out blue defense sunscreen

| **Phase** | **INCI Name** | **Wt.-%** |
|---|---|---|
| | Butyl methoxydibenzoylmethane | 1.00 |
| | Polysilicone-15 | 1.00 |
| | Ethylhexyl salicylate | 4.00 |
| | Octocrylene | 1.00 |
| | Titanium dioxide, Silica, dimethicone (PARSOL TX) | 1.50 |
| A | Potassium cetyl phosphate | 2.00 |
| | Diisopropyl sebacate | 3.00 |
| | Dimethicone | 2.00 |
| | Dicaprylyl ether | 2.00 |
| | Stearyl alcohol | 1.50 |
| | Hydroxyethyl acrylate/ sodium acryloyldimethyl taurate copolymer | 0.50 |
| | Polyacrylate crosspolymer-6 | 0.50 |
| | Phenoxyethanol, Ethylhexylglycerin | 1.00 |
| | Silica (VALVANCE Touch 210) | 3.00 |
| | Water | Ad 100 |
| B | Propanediol | 5.00 |
| | Niacinamide | 3.00 |
| | Chlorphenesin | 0.20 |
| | Methylene bis-benzotriazolyl tetramethylbutylphenol | 6.00 |
| C | Extract according to example 1 | 1.00 |
| | Tocopherol | 0.50 |
| | Parfum | 0.20 |

### Example 6: In and out blue defense sunscreen

| | **INCI name** | **Wt.-%** |
|---|---|---|
| A | Bis-ethylhexyloxyphenol methoxyphenyl triazine | 1.50 |
| | Polysilicone-15 | 1.00 |
| | Butyl methoxydibenzoylmethane | 2.00 |
| | Ethylhexyl salicylate | 5.00 |
| | Octocrylene | 1.50 |
| | Titanium dioxide, Silica, dimethicone (PARSOL TX) | 3.00 |
| | Potassium cetyl phosphate | 2.00 |
| | Diisopropyl sebacate | 3.00 |
| | Dimethicone | 2.00 |
| | Dicaprylyl ether | 2.00 |
| | Stearyl alcohol | 1.20 |
| | Hydroxyethyl acrylate/ sodium acryloyldimethyl taurate copolymer | 0.50 |
| | Polyacrylate crosspolymer-6 | 0.50 |
| | Phenoxyethanol, Ethylhexylglycerin | 1.00 |
| | Silica (VALVANCE Touch 210) | 3.00 |
| B | Water | Ad 100 |
| | Propanediol | 5.00 |
| | Niacinamide | 3.00 |
| | Chlorphenesin | 0.20 |
| C | Methylene bis-benzotriazolyl tetramethylbutylphenol | 8.00 |
| | Extract according to example 1 | 1.00 |
| | Tocopherol | 0.50 |
| | Parfum | 0.20 |

## Claims

1. A cosmetic composition comprising a scenedesmus rubescens extract and a cosmetically acceptable carrier.

2. The cosmetic composition according to claim 1, wherein the scenedesmus rubescens extract is either an aqueous or a dry extract of the biotechnologically produced microalgae *Scenedesmus Rubescens.*

3. The cosmetic composition according to claim 2, wherein the solid content in the aqueous scenedesmus rubens extract is selected in the range of 0.5 to 10 wt.-%, based on the total weight of the extract.

4. The cosmetic composition according to claim 2 or 3, wherein the pH of the aqueous scenedesmus rubescens extract is selected in the range of 3 to 6.

5. The cosmetic composition according to anyone of the preceding claims, wherein the scenedesmus rubescens extract is an aqueous extract having a solid content selected in the range of 1-5 wt.-% and which comprises an amount of phenoxyethanol selected in the range of 0.1 to 1 wt.-% and which exhibits a pH in the range of 4.5-5.5.

6. The cosmetic composition according to anyone of the preceding claims, wherein the amount of the scenedesmus rubescens extract (based on dry matter) in the cosmetic composition is selected in the range of 0.001 to 1 wt.-%, based on the total weight of the cosmetic composition.

7. The composition according to anyone of the preceding claims, wherein the composition is a skin care composition.

8. The composition according to anyone of the preceding claims, wherein the composition is in the form of O/W emulsions comprising an oily phase dispersed in an aqueous phase in the presence of an O/W emulsifier.

9. A composition according to claim 8, **characterized in that** the O/W emulsifier is potassium cetyl phosphate.

10. A composition according to any one of the preceding claims, **characterized in that** the composition further contains at least one UV-filter substance.

11. A method of protecting human skin against damage upon exposure to electromagnetic radiation, said method comprising the step of applying a cosmetic composition containing an effective amount of a scenedesmus rubescens extract to the skin.

12. A method according to claim 11, wherein the electromagnetic radiation is visible light, preferably blue light.

13. A method of reducing the generation of reactive oxygen species and/ or the formation of carbonylated proteins in humans when exposed to blue light, said method comprising the step of applying a cosmetic composition containing an effective amount of a scenedesmus rubescens extract to the skin.

14. A method according to any one of claims 11 to 13, wherein the effective amount of the scenedesmus rubescens extract (based on dry matter) in the cosmetic composition is selected in the range of 0.001 to 1 wt.-%, based on the total weight of the cosmetic composition.

15. Use of a scenedesmus rubescens extract to protect human skin against the adverse effects of electromagnetic radiation.
